# EUROPÄISCHE PATENTANMELDUNG

(11) **EP 4 230 249 A1**
(43) Veröffentlichungstag der Anmeldung: **23.08.2023**
(21) Anmeldenummer: 23156303.2
(22) Anmeldetag: 13.02.2023
(51) Int. Cl.: A61M 25/01, A61B 17/122, A61M 25/02, A61B 17/34

(54) **VORRICHTUNG ZUR UNTERSTÜTZUNG BEI DER PLATZIERUNG EINES NABELKATHETERS BEI FRÜH- UND NEUGEBORENEN**

(30) Priorität: 16.02.2022 IT 202200002873
(71) Anmelder: PFM Medical AG, 50996 Köln (DE)
(72) Erfinder: Welfers, Pia, 41844 Wegberg (DE); Haase, Bianca, 72070 Tübingen (DE); Kox, Georg, 41844 Wegberg (DE); Rauch, Daniel, 45147 Essen (DE)
(74) Vertreter: Hohendorf Kierdorf Patentanwälte PartGmbB

(57) **Zusammenfassung**

Die Erfindung betrifft eine Vorrichtung (1) zur Unterstützung bei der Platzierung eines Nabelkatheters (2) bei Früh- und Neugeborenen (3), umfassend:
einen ersten Schenkel (4) und einen zweiten Schenkel (5),
wobei der erste und zweite Schenkel (4, 5) im Bereich eines ersten Endes (6) jeweils eine halbzylindrische Ausbuchtung (7) umfassen und im Bereich des zweiten Endes (8) über ein Federelement (9) miteinander verbunden sind,
wobei das Federelement (9) den ersten und zweiten Schenkel (4, 5) durch eine Federkraft in einem ersten Betriebszustand hält, in welchem die jeweiligen ersten Enden (6) des ersten und zweiten Schenkels (4, 5) voneinander beabstandet sind und
wobei der erste und zweite Schenkel (4, 5) gegen die Federkraft in einen zweiten Betriebszustand überführt werden können, in welchem die jeweiligen ersten Enden (6) des ersten und zweiten Schenkels (4, 5) unmittelbar benachbart zueinander angeordnet sind,
wobei die halbzylindrischen Ausbuchtungen (7) im Bereich des jeweiligen ersten Endes (6) des ersten und zweiten Schenkels (4, 5) in dem zweiten Betriebszustand zusammen eine zylindrische Aufnahme (10) für die Nabelschnur (11) des Früh- und Neugeborenen (3) ausbilden.

## Beschreibung

Die Erfindung betrifft eine Vorrichtung zur Unterstützung bei der Platzierung eines Nabelkatheters bei Früh- und Neugeborenen, insbesondere in ein Nabelschnurgefäß eines Früh- und Neugeborenen.

Bei der Erstversorgung eines Früh- und Neugeborenen oder Frühgeborenen kann es vorkommen, dass (umgehend) ein Gefäßzugang benötigt wird, um dem Früh- und Neugeborenen Medikamente oder Flüssigkeiten verabreichen zu können. Von besonderer Wichtigkeit ist insbesondere, dass die Erstversorgung des Früh- und Neugeborenen im Rahmen einer Reanimation besonders schnell und sicher erfolgen muss.

Im Stand der Technik sind insbesondere drei unterschiedliche Möglichkeiten des Gefäßzugangs bekannt, welche regelmäßig zum Einsatz kommen: der Nabelkatheter, der periphere Venenzugang und der intraossäre Zugang (i.o.-Zugang). Alle Zugänge werden durch erfahrenes Personal gelegt. Dabei bedarf es viel Übung und Erfahrung der behandelnden Personen. Insbesondere bei der erstgenannten Alternative, sind mehrere Personen zeitgleich damit beschäftigt, den Zugang zu legen. Dies führt häufig zu Platzproblemen rund um das Früh- und Neugeborene.

Die rasche Anlage eines peripheren Venenkatheters (PVK) erfolgt durch besonders geschultes und erfahrenes Personal. Dies gelingt jedoch nicht immer und ist umso schwieriger, je unreifer oder deprimierter das Früh- und Neugeborene ist. insbesondere während einer Reanimation gestaltet sich die Anlage des Katheters oftmals besonders schwierig. Als Punktionsstellen kommen der Handrücken, der Fußrücken, die Kopfvenen sowie die Cubitalvenen in Frage. Insbesondere bei sehr kleinen Frühgeborenen und Früh- und Neugeborenen in einer schlechten Kreislaufsituation ist das Legen von peripheren venösen Zugängen meist nicht möglich.

Als alternativen Zugangsweg kann ein i.o.-Zugang über die proximale Tibia gelegt werden. Der i.o.-Zugang ist allerdings erst ab einem Gewicht von 3000 Gramm zulässig und birgt zusätzlich verschiedene Risiken, wie Knochenverletzungen, Entzündungen und Wachstumsstörungen des Unterschenkelknochens. Allerdings ist zum aktuellen Zeitpunkt eine Anlage eines i.o.-Zugangs im Vergleich zu einem Nabelkatheter technisch einfacher zu bewerkstelligen und gelingt somit oftmals deutlich schneller.

Ein weiterer alternativer Zugangsweg ist der Nabelkatheter. Hierbei wird ein Blutgefäß der Nabelschnur mit einem Katheter versehen bzw. kanüliert. Bei dem Blutgefäß kann es sich um eine Nabelvene oder eine Nabelarterie handeln. Ein venöser oder arterieller Zugang in den ersten Lebensminuten beim kritisch kranken Früh- und Neugeborenen ist essenziell, um Volumenboli, Glukose und Notfallmedikamente wie Suprarenin zu verabreichen. Gelingt dies nicht, so ist mit Folgeschäden durch Hypotonie, Hypoglykämie oder anhaltender Bradykardie zu rechnen.

Das größte Problem beim Legen eines Nabelvenenkatheters (NVK) oder Nabelarterienkatheters (NAK) ist, die Blutgefäße zu detektieren und so zu präparieren, dass das Gefäßlumen sichtbar wird. Dies wird im aktuellen Stand der Technik beispielsweise gelöst durch das händische Spreizen des Nabels mittels zweier chirurgischer Pinzetten. Hierfür ist zumindest eine weitere Person notwendig, die den Nabel derart präsentiert, dass eine zweite Person den Katheter legen kann.

Somit gibt es zwei große Nachteile beim Legen eines Nabelkatheters. Zum einen bedarf es mindestens zwei erfahrener Ärzte, welche aufeinander abgestimmt sind, einen Katheter zu legen. Diese Ärzte arbeiten beide aktiv am Nabel, sodass es zu Platzproblemen rund um das Früh- und Neugeborene kommen kann. Zum anderen bedarf es viel Erfahrung der behandelnden Ärzte. Das Legen eines Katheters gestaltet sich meist als handwerklich sehr schwierig oder endet frustran.

Um diese beiden Nachteile zu überwinden, offenbart die WO 2018/202844 A1 eine medizinische Vorrichtung zur Legung eines Katheters in ein Nabelschnurblutgefäß eines Früh- und Neugeborenen, wobei die medizinische Vorrichtung eine Längsachse, ein proximales und ein distales Ende aufweist. Die vorgeschlagene Vorrichtung ist als ein Mantel ausgestaltet, um einen Teil einer Nabelschnur zu umschließen. Die medizinische Vorrichtung umfasst eine Abklemmvorrichtung, um die Nabelschnur abzuklemmen, eine Abtrennvorrichtung, um die Nabelschnur zur Bildung einer Nabelschnittfläche zu durchtrennen, und eine Einhakvorrichtung, um die Nabelschnittfläche aufzuspreizen. Die medizinische Vorrichtung klemmt die Nabelschnur ab, trennt die Nabelschnur und spreizt diese zum Legen des Nabelkatheters auf. Nachteilig an der medizinischen Vorrichtung ist jedoch der komplexe Aufbau und die damit verbundenen Herstellungskosten. Zwar ist die medizinische Vorrichtung wiederverwendbar, muss aber nach jedem Einsatz desinfiziert werden, was insbesondere aufgrund der Vielzahl von beweglichen Elementen sehr aufwendig ist. Ein weiterer Nachteil ist, dass die Lage und anatomische Ausprägung der Nabelgefäße sehr stark variiert und daher die im obigen Patent beschriebene Lösung kaum umgesetzt werden kann.

Vor diesem Hintergrund ist es die Aufgabe der vorliegenden Erfindung, eine neue medizinische Vorrichtung zur Platzierung eines Katheters in ein Nabelschnurblutgefäß zu offenbaren, welches das schwierige Einführen eines Katheters über ein Nabelschnurblutgefäß vereinfacht und die Nachteile der aus dem Stand der Technik bekannten medizinischen Vorrichtungen vermeidet.

Die Aufgabe wird erfindungsgemäß gelöst durch eine Vorrichtung zur Unterstützung bei der Platzierung eines Nabelkatheters bei Früh- und Neugeborenen, umfassend:
einen ersten Schenkel und einen zweiten Schenkel,
wobei der erste und zweite Schenkel im Bereich eines ersten Endes jeweils eine halbzylindrische Ausbuchtung umfassen und im Bereich des zweiten Endes über ein Federelement miteinander verbunden sind,
wobei das Federelement den ersten und zweiten Schenkel durch eine Federkraft in einem ersten Betriebszustand hält, in welchem die jeweiligen ersten Enden des ersten und zweiten Schenkels voneinander beabstandet sind und wobei der erste und zweite Schenkel gegen die Federkraft in einen zweiten Betriebszustand überführt werden können, in welchem die jeweiligen ersten Enden des ersten und zweiten Schenkels unmittelbar benachbart zueinander angeordnet sind,
wobei die halbzylindrischen Ausbuchtungen im Bereich des jeweiligen ersten Endes des ersten und zweiten Schenkels in dem zweiten Betriebszustand zusammen eine zylindrische Aufnahme für die Nabelschnur des Früh- und Neugeborenen ausbilden.

Die erfindungsgemäße Vorrichtung hat einen besonders einfachen Aufbau, da lediglich zwei bewegliche Schenkel über ein Federelement miteinander verbunden sind. Die Nabelschnur wird in dem zweiten Betriebszustand in der zylindrischen Aufnahme fixiert. Die distalen Kanten des ersten und zweiten Schenkels, die im zweiten Betriebszustand benachbart zueinander sind, dienen dabei als Führung für das Skalpell, um die Nabelschnur an der distalen Kante der erfindungsgemäßen Vorrichtung abzutrennen. Dadurch wird eine besonders gerade Schnittkante erzielt und es hat sich herausgestellt, dass bei einer derartigen Schnittkante auf das aus dem Stand der Technik erforderliche Aufspreizen der Nabelschnur verzichtet werden kann, da die Gefäße in der Nabelschnur klar zu erkennen sind und auch der Katheter problemlos eingeführt werden kann.

Mittels der erfindungsgemäßen Vorrichtung lässt sich einfach und schnell ein Nabelkatheter in die Gefäße der Nabelschnur eines Früh- und Neugeborenen einführen. Dazu wird die Nabelschnur im ersten Betriebszustand so zwischen dem ersten und zweiten Schenkel der Vorrichtung angeordnet, dass diese beim Zusammendrücken, als Überführen in den zweiten Betriebszustand, in der Aufnahme am ersten Ende des ersten und zweiten Schenkels angeordnet ist. Sobald die Nabelschnur im zweiten Betriebszustand in der ausgebildeten zylindrischen Aufnahme fixiert ist, kann diese mit einem Skalpell abgeschnitten werden, wobei das Skalpell an der distalen Kante der erfindungsgemäßen Vorrichtung geführt wird, wodurch ein sauberer Schnitt erzielt wird. Nachfolgend kann der Katheter in das gewünschte Gefäß der Nabelschnur des Früh- und Neugeborenen eingeführt werden. Wird nun die Vorrichtung losgelassen, nimmt diese automatisch den ersten Betriebszustand an, in welchem der erste Schenkel und der zweite Schenkel gespreizt sind, so dass die Nabelschnur freigegeben wird. Um einen Austritt von Blut aus den nicht katheterisierten Gefäßen zu vermeiden, kann die Nabelschnur abgebunden werden. Dadurch wird gleichzeitig der Katheter in dem abgeklemmten Gefäß fixiert, so dass dieser nicht mehr verschoben werden kann.

Die erfindungsgemäße Vorrichtung weist einen besonders einfachen Aufbau auf, insbesondere ohne bewegliche Teile wie verschwenkbare Messer oder Haken. Dadurch lässt sich die erfindungsgemäße Vorrichtung sehr einfach sterilisieren. Alternativ kann die erfindungsgemäße Vorrichtung aber auch problemlos als Einmalprodukt hergestellt werden und nach der Verwendung entsorgt werden, da der einfache Aufbau eine kostengünstige Herstellung ermöglicht. Die Nachteile des Standes der Technik werden somit vermieden.

In einer vorteilhaften Variante der Erfindung umfasst die Vorrichtung eine Arretiervorrichtung, zur Arretierung der Vorrichtung in dem zweiten Betriebszustand. Dadurch muss der Anwender der Vorrichtung diese nicht entgegen der Federkraft in dem zweiten Betriebszustand halten, sondern die Vorrichtung ist in diesem zweiten Betriebszustand arretiert. Der Anwender kann sich somit voll auf die Abtrennung der Nabelschnur und das Legen des Nabelkatheters konzentrieren. Zweckmäßigerweise arretiert sich die Arretiervorrichtung automatisch, wenn der Anwender die Vorrichtung in den zweiten Betriebszustand überführt hat.

Gemäß einer zweckmäßigen Variante der Erfindung ist die Arretiervorrichtung lösbar ausgebildet, insbesondere als Rastverbindung, um die Vorrichtung nach dem Legen des Nabelkatheters wieder entfernen zu können. Dazu wird beispielsweise die Rastverbindung durch Bewegen der Raste gelöst.

Nach einer erfindungsgemäßen Variante ist die Arretiervorrichtung am ersten Ende des ersten und zweiten Schenkels ausgebildet, insbesondere ein erster Teil der Arretiervorrichtung am ersten Ende des ersten Schenkels und ein zweiter Teil der Arretiervorrichtung am ersten Ende des zweiten Schenkels. Somit werden insbesondere die ersten Enden des ersten und zweiten Schenkels durch die Arretiervorrichtung sicher relativ zueinander fixiert, genau in dem Bereich, in welchem die zylindrische Aufnahme für die Nabelschnur im zweiten Betriebszustand ausgebildet wird. Die Nabelschnur wird daher sicher in der Aufnahme fixiert, da sich die ersten Enden des ersten und zweiten Schenkels aufgrund der Arretiervorrichtung nicht bewegen können. Beispielsweise weist das erste Ende des ersten Schenkels eine Öse auf und das erste Ende des zweiten Schenkels eine Raste (Vorsprung), welche im zweiten Betriebszustand in die Öse eingreift.

In einer besonders bevorzugten Variante der Erfindung umfasst die Vorrichtung eine Erhöhung auf der distalen Seite der Vorrichtung im Bereich der zylindrischen Aufnahme der Nabelschnur im zweiten Betriebszustand. Diese Erhöhung dient als Schnittkante (Gegenlager) beim Durchtrennen der Nabelschnur. Die Nabelschnur wird mittels eines Skalpells durchtrennt, welches entlang der distalen Kante der Vorrichtung geführt wird. In Schnittrichtung hinter der Nabelschnur ist die Erhöhung angeordnet, so dass die Nabelschnur beim Durchtrennen durch die Erhöhung gestützt wird, wodurch sich diese leichter durchtrennen lässt.

Gemäß einer zweckmäßigen Variante der Erfindung ist die Erhöhung zweigeteilt und ein Teil der Erhöhung ist jeweils auf dem ersten und zweiten Schenkel angeordnet. Im zweiten Betriebszustand sind die beiden Teile der Erhöhung unmittelbar benachbart zueinander angeordnet. Üblicherweise wird ein Schnitt aus Sicherheitsgründen immer vom Körper des Anwenders weggeführt. In diesem Fall bedeutet das, der Schnitt zur Durchtrennung der Nabelschnur wird in Richtung des ersten Endes der Vorrichtung durchgeführt. Die Erhöhung muss also am ersten Ende der Vorrichtung angeordnet sein, vorzugsweise in Schnittrichtung nach der zylindrischen Aufnahme für die Nabelschnur. In diesem Bereich sind der erste und zweite Schenkel im ersten Betriebszustand jedoch voneinander getrennt, damit die Nabelschnur in der Vorrichtung aufgenommen werden kann. Aus diesem Grund ist es zweckmäßig, wenn jeder Schenkel einen Teil der Erhöhung aufweist, welche zusammen im zweiten Betriebszustand die Erhöhung ausbilden.

In einer vorteilhaften Variante der Erfindung ist am distalen Ende des jeweiligen Teils der Erhöhung ein halbkreisförmiges Element angeordnet ist, welches axial mit der halbzylindrischen Ausbuchtung des jeweiligen ersten und zweiten Schenkels ausgerichtet ist, wobei die beiden halbkreisförmigen Elemente in dem zweiten Betriebszustand eine kreisförmige Aufnahme ausbilden, um die Nabelschnur des Früh- und Neugeborenen zu fixieren, wobei die kreisförmige Aufnahme in dem zweiten Betriebszustand axial zu der zylindrischen Aufnahme ausgerichtet ist und von dieser beabstandet ist, so dass zwischen der zylindrischen Aufnahme und der kreisförmigen Aufnahme ein Schnittkanal verbleibt. Dadurch wird die Nabelschnur des Früh- und Neugeborenen sowohl unterhalb des Schnittkanals durch die zylindrische Aufnahme als auch oberhalb des Schnittkanals durch die kreisförmige Aufnahme fixiert. Dadurch lässt sich die Nabelschnur besonders einfach durchtrennen.

Gemäß einer erfindungsgemäßen Variante ist die Erhöhung oder sind die Teile der Erhöhung über eine Sollbruchkante mit dem ersten und/oder zweiten Schenkel der Vorrichtung verbunden. Die Erhöhung kann also nach dem Durchtrennen der Nabelschnur einfach von der Vorrichtung getrennt werden, indem diese einfach abgeknickt wird. Dadurch wird verhindert, dass die Erhöhung oder damit verbundene Teile wie beispielsweise die halbkreisförmigen Elemente die Einführung des Katheters in ein Gefäß der Nabelschnur behindern.

Nach einer weiteren Variante der Erfindung sind die halbzylindrischen Ausbuchtungen im ersten Betriebszustand und die zylindrische Aufnahme im zweiten Betriebszustand konisch ausgebildet, wobei sich der Konus von der proximalen Seite zu der distalen Seite der Vorrichtung erweitert. Dadurch wird die Nabelschnur besonders gut in der zylindrischen Aufnahme fixiert. Ferner können dadurch beispielsweise die Gefäße in der Nabelschnur im proximalen Bereich, also dem engeren Bereich des Konus, abgedrückt werden, so dass nach dem Durchtrennen kein oder nur sehr wenig Blut aus den Gefäßen der Nabelschnur austreten kann. Der Konus sollte dabei so ausgebildet sein, dass der Katheter ausreichend tief in das Gefäß der Nabelschnur eingeführt werden kann, um einen Zugang zum Gefäßsystem des Früh- und Neugeborenen herzustellen.

Gemäß einer erfindungsgemäßen Variante sind die halbzylindrischen Ausbuchtungen im ersten Betriebszustand und die zylindrische Aufnahme im zweiten Betriebszustand auf der Innenseite gerillt, insbesondere weise diese Durchbrechungen zur Ausbildung von Lamellen auf. Dadurch wird die Fixierung der Nabelschnur in der zylindrischen Aufnahme nochmals verbessert.

In einer erfindungsgemäßen Variante weisen der erste und zweite Schenkel an der distalen Seite jeweils sich nach außen erstreckende Flügel als Fingerschutz auf. Diese schützen die Finger des Anwenders der Vorrichtung beim Durchtrennen der Nabelschnur vor Schnittverletzungen.

Gemäß einer zweckmäßigen Variante der Erfindung ist das Federelement kreisförmig ausgebildet, welches im ersten Betriebszustand geöffnet ist und im zweiten Betriebszustand geschlossen ist. Auf diese Art und Weise lässt sich das Federelement einfach herstellen und in die Vorrichtung integrieren. Durch den Durchmesser des kreisförmigen Federelements kann die Federkraft vorab eingestellt werden.

Nach einer vorteilhaften Variante ist die erfindungsgemäße Vorrichtung einstückig ausgebildet. Eine einstückige Vorrichtung hat den Vorteil, dass diese einfach desinfiziert werden kann. insbesondere lassen sich derartige einfach einstückige Vorrichtungen kostengünstig herstellen, so dass diese vorzugsweise als Einwegprodukt ausgebildet sind, da die Herstellung günstiger ist als ein Desinfizieren der Vorrichtung.

Zweckmäßigerweise ist die erfindungsgemäße Vorrichtung aus einem Kunststoff hergestellt.

Zur besseren Handhabung können der erste und zweite Schenkel Griffflächen aufweisen, welche beispielsweise gerillt sind. Dadurch verbessert sich die Griffigkeit.

Vorzugsweise werden die erfindungsgemäßen Vorrichtungen mit unterschiedlichen Durchmessern der zylindrischen Aufnahme im zweiten Betriebszustand hergestellt und bevorratet, damit im Ernstfall schnell eine Vorrichtung mit entsprechendem Durchmesser für die aktuelle Nabelschnur ausgewählt werden kann. Beispielsweise weist die zylindrische Aufnahme im zweiten Betriebszustand einen Durchmesser von 5 mm bis 15 mm, insbesondere 7 mm, 9 mm oder 11 mm auf.

Nachfolgend wird die Erfindung anhand von in den Figuren dargestellten Ausführungsbeispielen näher erläutert. Es zeigen:
- Fig. 1: eine perspektivische Ansicht einer Ausführungsform einer erfindungsgemäßen Vorrichtung zur Unterstützung bei der Platzierung eines Nabelkatheters bei Früh- und Neugeborenen im ersten Betriebszustand,
- Fig. 2: eine Draufsicht auf die Vorrichtung aus Fig. 1,
- Fig. 3: eine perspektivische Ansicht einer Ausführungsform einer erfindungsgemäßen Vorrichtung im zweiten Betriebszustand mit fixierter Nabelschnur vor dem Durchtrennen,
- Fig. 4: eine perspektivische Ansicht der Vorrichtung aus Fig. 3 nach dem Durchtrennen der Nabelschnur und Entfernen der Erhöhung, und
- Fig. 5: einen gelegten Nabelkatheter nach dem Entfernen der erfindungsgemäßen Vorrichtung.

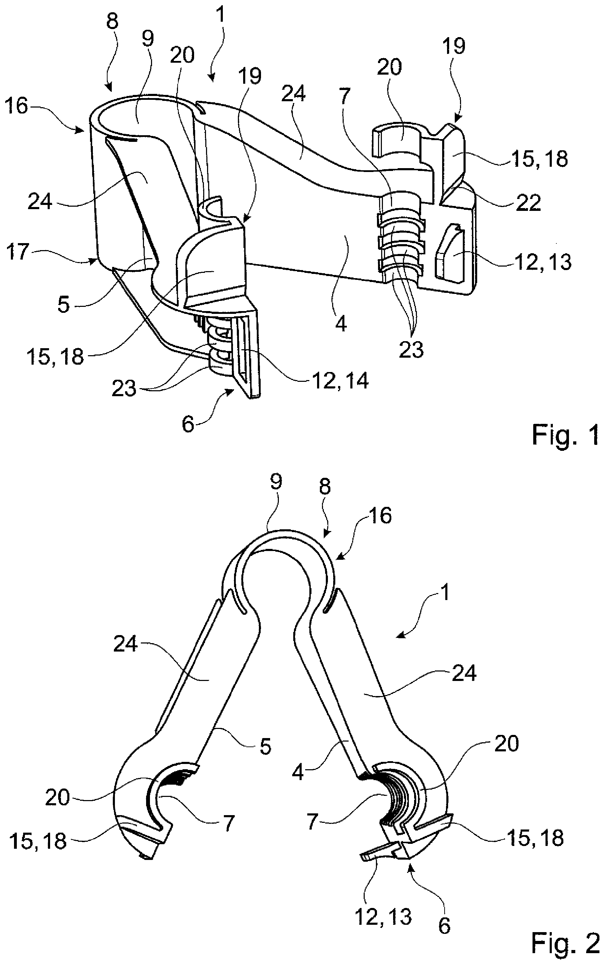

Fig. 1 zeigt eine perspektivische Ansicht einer Ausführungsform einer erfindungsgemäßen Vorrichtung 1 zur Unterstützung bei der Platzierung eines Nabelkatheters 2 bei Früh- und Neugeborenen 3. Fig. 2 zeigt eine Draufsicht auf die Vorrichtung 1 aus Fig. 1. Die Vorrichtung 1 umfasst einen ersten Schenkel 4 und einen zweiten Schenkel 5. Der erste Schenkel 4 und der zweite Schenkel 5 weisen jeweils im Bereich eines ersten Endes 6 eine halbzylindrische Ausbuchtung 7 auf. Im Bereich eines zweiten Endes 8 sind der erste Schenkel 4 und der zweite Schenkel 5 über ein Federelement 9 miteinander verbunden.

Das Federelement 9 hält den ersten Schenkel 4 und den zweiten Schenkel 5 durch eine ihm innewohnende Federkraft in einem ersten Betriebszustand, in welchem die jeweiligen ersten Enden 6 des ersten Schenkels 4 und des zweiten Schenkels 5 voneinander beabstandet sind. Fig. 1 zeigt die Vorrichtung 1 in dem ersten Betriebszustand.

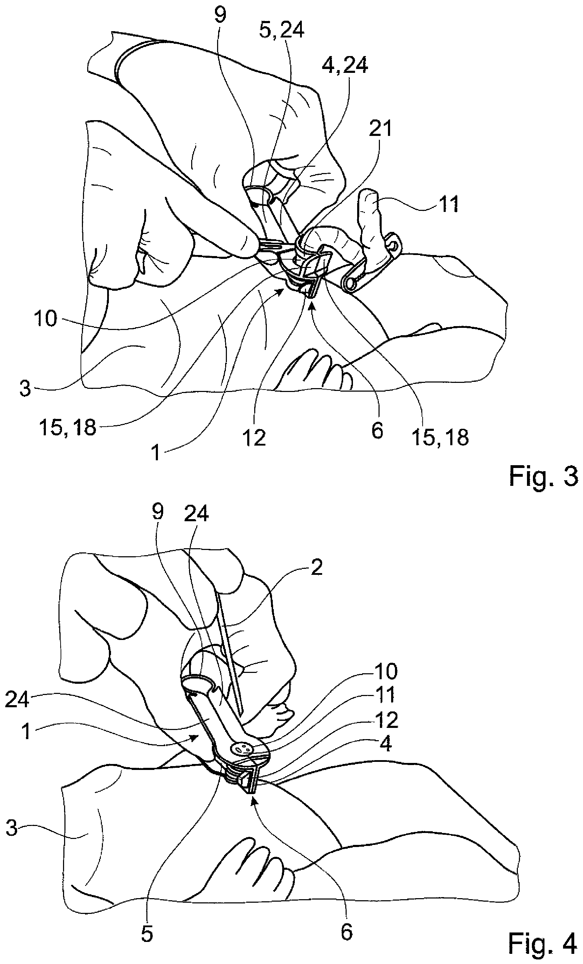

Der erste Schenkel 4 und der zweite Schenkel 5 können gegen die Federkraft in einen zweiten Betriebszustand überführt werden, in welchem die jeweiligen ersten Enden 6 des ersten Schenkels 4 und des zweiten Schenkels 5 unmittelbar benachbart zueinander angeordnet sind. Die Figuren 3 und 4 zeigen beispielsweise eine erfindungsgemäße Vorrichtung 1 in dem zweiten Betriebszustand.

Die Vorrichtung 1 umfasst gemäß dem ersten Ausführungsbeispiel aus Fig. 1 eine Arretiervorrichtung 12, zur Arretierung der Vorrichtung 1 in dem zweiten Betriebszustand. Die Arretiervorrichtung 12 ist in dem ersten Ausführungsbeispiel als lösbare Rastverbindung ausgebildet. Die als Rastverbindung ausgebildete Arretiervorrichtung ist am ersten Ende 6 des ersten Schenkels 4 und zweiten Schenkels 5 angeordnet, wobei ein erster Teil 13 der Arretiervorrichtung 12 am ersten Ende 6 des ersten Schenkels 4 und ein zweiter Teil 14 der Arretiervorrichtung 12 am ersten Ende 6 des zweiten Schenkels 5 angeordnet ist. Gemäß dem dargestellten Ausführungsbeispiel ist am ersten Ende 6 des ersten Schenkels 4 ein Vorsprung (Raste) als erster Teil 13 ausgebildet, welcher in eine Öffnung (Öse) am ersten Ende 6 des zweiten Schenkels 5 eingreifen kann, wobei die Öffnung den zweiten Teil 14 der Arretiervorrichtung 12 ausbildet.

Die erfindungsgemäße Vorrichtung aus Fig. 1 umfasst ferner eine Erhöhung 15 auf der distalen Seite 16 der Vorrichtung 1 im Bereich der zylindrischen Aufnahme 10 im zweiten Betriebszustand. Die distale Seite im Sinne der Erfindung ist dabei die vom Früh- und Neugeborenen 3 abgewandte Seite der Vorrichtung 1 und die proximale Seite ist die dem Früh- und Neugeborenen 3 zugewandte Seite der Vorrichtung 1.

Die Erhöhung 15 ist im Bereich der zylindrischen Aufnahme 10 im zweiten Betriebszustand bzw. der halbzylindrischen Ausbuchtungen 7 im ersten Betriebszustand angeordnet. Gemäß dem in Fig. 1 dargestellten ersten Ausführungsbeispiel ist die Erhöhung zweigeteilt ausgebildet, wobei ein Teil der Erhöhung 18 jeweils auf dem ersten und zweiten Schenkel 4, 5 angeordnet ist. Im zweiten Betriebszustand der Vorrichtung 1 sind die beiden Teile 18 der Erhöhung 15 unmittelbar benachbart zueinander angeordnet.

Am distalen Ende 19 des jeweiligen Teils 18 der Erhöhung 15 ist ein halbkreisförmiges Element 20 angeordnet. Das halbkreisförmige Element 20 ist axial mit der halbzylindrischen Ausbuchtung 7 des jeweiligen ersten und zweiten Schenkels 4, 5 ausgerichtet. In dem zweiten Betriebszustand bilden die beiden halbkreisförmigen Elemente 20 eine kreisförmige Aufnahme 21 aus, um die Nabelschnur 11 des Früh- und Neugeborenen 3 zu fixieren. Die kreisförmige Aufnahme 21 ist in dem zweiten Betriebszustand axial zu der zylindrischen Aufnahme 10 ausgerichtet und von dieser beabstandet, so dass zwischen der zylindrischen Aufnahme 10 und der kreisförmigen Aufnahme 21 ein Schnittkanal verbleibt.

Zweckmäßigerweise ist die Erhöhung 15 oder sind die Teile der Erhöhung 18 über eine Sollbruchkante 22 mit dem ersten und/oder zweiten Schenkel 4, 5 der Vorrichtung 1 verbunden. Dadurch kann die Erhöhung 15 schnell und einfach von der Vorrichtung 1 durch Knicken entfernt werden, damit diese die Einführung des Nabelkatheters 2 in ein Gefäß der Nabelschnur 11 nicht behindert.

Vorteilhafterweise sind die halbzylindrischen Ausbuchtungen 7 im ersten Betriebszustand und die zylindrische Aufnahme 10 im zweiten Betriebszustand konisch ausgebildet, wobei sich der Konus von der proximalen Seite 17 zu der distalen Seite 16 der Vorrichtung 1 erweitert. Dadurch wird die Nabelschnur 11 besonders gut in der erfindungsgemäßen Vorrichtung 1 fixiert. Insbesondere können so die Gefäße der Nabelschnur 11 abgedrückt werden, damit nach dem Durchtrennen kein Blut austritt, aber gleichzeitig der Nabelkatheter 2 in das Gefäß der Nabelschnur 11 eingeführt werden kann.

Zur weiteren Verbesserung der Fixierung der Nabelschnur 11 in der zylindrischen Aufnahme 10 der Vorrichtung 1 im zweiten Betriebszustand weisen die halbzylindrischen Ausbuchtungen 7 bzw. die zylindrische Aufnahme 10 Durchbrechungen auf, zur Ausbildung von Lamellen 23.

Ferner weisen der erste Schenkel 4 und der zweite Schenkel 5 jeweils an der distalen Seite 16 einen sich nach außen erstreckenden Flügel 24 als Fingerschutz auf.

Wie insbesondere der Fig. 2 entnommen werden kann, ist das Federelement 9 kreisförmig ausgebildet, welches im ersten Betriebszustand geöffnet ist. Wie der Fig. 3 entnommen werden kann, ist das kreisförmige Federelement 9 im zweiten Betriebszustand geschlossen.

Zweckmäßigerweise ist die Vorrichtung 1 einstückig ausgebildet und bevorzugt aus einem Kunststoff hergestellt. Dies ermöglicht eine kostengünstige Herstellung, so dass die Vorrichtung 1 insbesondere als Einmalprodukt angeboten werden kann.

Die Figuren 3 bis 5 zeigen die Verwendung einer erfindungsgemäßen Vorrichtung 1 zum Legen eines Nabelkatheters 2 bei Früh- und Neugeborenen 3. In dem ersten Betriebszustand der Vorrichtung 1 wird die Nabelschnur des Früh- und Neugeborenen 3 zwischen dem ersten Schenkel 4 und dem zweiten Schenkel 5 im Bereich der beiden halbzylindrischen Ausbuchtungen 7 angeordnet. Sodann wird die Vorrichtung 1 in den zweiten Betriebszustand überführt, indem der Nutzer den ersten Schenkel 4 und den zweiten Schenkel 5 der Vorrichtung 1 aufeinander zu bewegt. Sobald der erste und zweite Schenkel 4, 5 unmittelbar benachbart zueinander sind, wird die Vorrichtung 1 in diesem zweiten Betriebszustand durch die Arretiervorrichtung 12 in dem zweiten Betriebszustand fixiert. Fig. 3 zeigt diesen zweiten Betriebszustand.

Der Nutzer kann nun die Nabelschnur 11 durchtrennen. Dazu wird das Skalpell 25 entlang der distalen Seite der Vorrichtung 1 geführt, wobei die distale Kante der Vorrichtung 1 als Führung dient. Die Nabelschnur 11 wird durch die kreisförmige Aufnahme 21 und die zylindrische Aufnahme 10 fixiert. Die Nabelschnur 11 wird gegen die Erhöhung 15 durchtrennt.

Nachfolgend wird die Erhöhung 15 abgeknickt, so dass die durchtrennte Nabelschnur 11, insbesondere deren Gefäße, gut sichtbar und zugänglich ist. Dieser Zustand ist in Fig. 4 dargestellt. Der Nabelkatheter 2 kann nun schnell und problemlos in das gewünschte Gefäß der Nabelschnur 11 eingeführt werden.

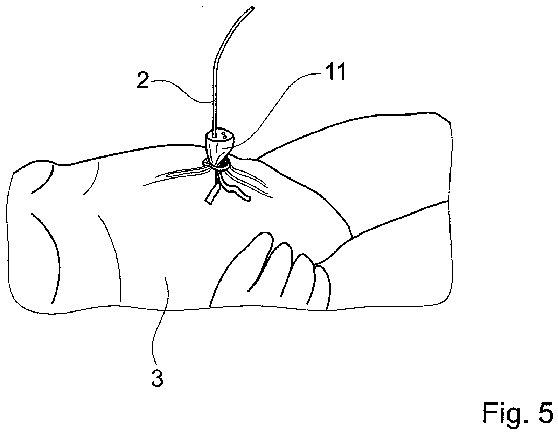

Wie in Fig. 5 dargestellt, wird die Vorrichtung 1 nach dem Legen des Nabelkatheters 2 durch Lösen der Arretiervorrichtung 12 entfernt. Um einen Blutaustritt aus den nicht katheterisierten Gefäßen der Nabelschnur zu verhindern, wird diese abgebunden. Dabei sollte darauf geachtet werden, dass der gelegte Nabelkatheter 2 offen bleibt.

### Bezugszeichenliste

- 1: Vorrichtung
- 2: Nabelkatheter
- 3: Früh- und Neugeborenes
- 4: erster Schenkel
- 5: zweiter Schenkel
- 6: erstes Ende
- 7: halbzylindrische Ausbuchtung
- 8: zweites Ende
- 9: Federelement
- 10: zylindrische Aufnahme
- 11: Nabelschnur
- 12: Arretiervorrichtung
- 13: erster Teil der Arretiervorrichtung
- 14: zweiter Teil der Arretiervorrichtung
- 15: Erhöhung
- 16: distale Seite (Vorrichtung)
- 17: proximale Seite (Vorrichtung)
- 18: Teil der Erhöhung
- 19: distales Ende (Erhöhung)
- 20: halbkreisförmiges Element
- 21: kreisförmige Aufnahme
- 22: Sollbruchkante
- 23: Lamellen
- 24: Flügel (Fingerschutz)
- 25: Skalpell

## Patentansprüche

1. Vorrichtung (1) zur Unterstützung bei der Platzierung eines Nabelkatheters (2) bei Früh- und Neugeborenen (3), umfassend:
einen ersten Schenkel (4) und einen zweiten Schenkel (5),
wobei der erste und zweite Schenkel (4, 5) im Bereich eines ersten Endes (6) jeweils eine halbzylindrische Ausbuchtung (7) umfassen und im Bereich des zweiten Endes (8) über ein Federelement (9) miteinander verbunden sind,
wobei das Federelement (9) den ersten und zweiten Schenkel (4, 5) durch eine Federkraft in einem ersten Betriebszustand hält, in welchem die jeweiligen ersten Enden (6) des ersten und zweiten Schenkels (4, 5) voneinander beabstandet sind und wobei der erste und zweite Schenkel (4, 5) gegen die Federkraft in einen zweiten Betriebszustand überführt werden können, in welchem die jeweiligen ersten Enden (6) des ersten und zweiten Schenkels (4, 5) unmittelbar benachbart zueinander angeordnet sind,
wobei die halbzylindrischen Ausbuchtungen (7) im Bereich des jeweiligen ersten Endes (6) des ersten und zweiten Schenkels (4, 5) in dem zweiten Betriebszustand zusammen eine zylindrische Aufnahme (10) für die Nabelschnur (11) des Früh- und Neugeborenen (3) ausbilden.

2. Vorrichtung (1) nach Anspruch 1,
weiterhin umfassend eine Arretiervorrichtung (12), zur Arretierung der Vorrichtung (1) in dem zweiten Betriebszustand.

3. Vorrichtung (1) nach Anspruch 2,
wobei die Arretiervorrichtung (12) lösbar oder unlösbar ausgebildet ist, insbesondere als Rastverbindung.

4. Vorrichtung (1) nach Anspruch 2 oder Anspruch 3,
wobei die Arretiervorrichtung (12) am ersten Ende (6) des ersten und zweiten Schenkels (4, 5) ausgebildet ist, insbesondere ein erster Teil (13) der Arretiervorrichtung (12) am ersten Ende (6) des ersten Schenkels (4) und ein zweiter Teil (14) der Arretiervorrichtung (12) am ersten Ende (6) des zweiten Schenkels (5).

5. Vorrichtung (1) nach einem der Ansprüche 1 bis 4,
weiterhin umfassend eine Erhöhung (15) auf der distalen Seite (16) der Vorrichtung (1) im Bereich der zylindrischen Aufnahme (10) im zweiten Betriebszustand.

6. Vorrichtung (1) nach Anspruch 5,
wobei die Erhöhung (15) zweigeteilt ist und ein Teil der Erhöhung (18) jeweils auf dem ersten und zweiten Schenkel (4, 5) angeordnet ist, wobei die beiden Teile der Erhöhung (18) im zweiten Betriebszustand unmittelbar benachbart zueinander sind.

7. Vorrichtung (1) nach Anspruch 6,
wobei am distalen Ende (19) des jeweiligen Teils (18) der Erhöhung (15) ein halbkreisförmiges Element (20) angeordnet ist, welches axial mit der halbzylindrischen Ausbuchtung (7) des jeweiligen ersten und zweiten Schenkels (4, 5) ausgerichtet ist, wobei die beiden halbkreisförmigen Elemente (20) in dem zweiten Betriebszustand eine kreisförmige Aufnahme (21) ausbilden, um die Nabelschnur (11) des Früh- und Neugeborenen (3) zu fixieren, wobei die kreisförmige Aufnahme (21) in dem zweiten Betriebszustand axial zu der zylindrischen Aufnahme (10) ausgerichtet ist und von dieser beabstandet ist, so dass zwischen der zylindrischen Aufnahme (10) und der kreisförmigen Aufnahme (21) ein Schnittkanal verbleibt.

8. Vorrichtung (1) nach einem der Ansprüche 5 bis 7,
wobei die Erhöhung (15) oder die Teile der Erhöhung (18) über eine Sollbruchkante (22) mit dem ersten und/oder zweiten Schenkel (4, 5) der Vorrichtung (1) verbunden ist.

9. Vorrichtung (1) nach einem der Ansprüche 1 bis 8,
wobei die halbzylindrischen Ausbuchtungen (7) im ersten Betriebszustand und die zylindrische Aufnahme (10) im zweiten Betriebszustand konisch ausgebildet sind, wobei sich der Konus von der proximalen Seite (17) zu der distalen Seite (16) der Vorrichtung (1) erweitert.

10. Vorrichtung (1) nach einem der Ansprüche 1 bis 9,
wobei die halbzylindrischen Ausbuchtungen (7) im ersten Betriebszustand und die zylindrische Aufnahme (10) im zweiten Betriebszustand auf der Innenseite gerillt sind, insbesondere Durchbrechungen zur Ausbildung von Lamellen (23) aufweisen.

11. Vorrichtung (1) nach einem der Ansprüche 1 bis 10,
wobei der erste und zweite Schenkel (4, 5) an der distalen Seite (16) jeweils sich nach außen erstreckende Flügel (24) als Fingerschutz aufweisen.

12. Vorrichtung (1) nach einem der Ansprüche 1 bis 11,
wobei das Federelement (9) kreisförmig ausgebildet ist, welches im ersten Betriebszustand geöffnet ist und im zweiten Betriebszustand geschlossen ist.

13. Vorrichtung (1) nach einem der Ansprüche 1 bis 12,
wobei die Vorrichtung (1) einstückig ausgebildet ist.

14. Vorrichtung (1) nach einem der Ansprüche 1 bis 13,
wobei die Vorrichtung (1) aus einem Kunststoff hergestellt ist.
